# EUROPEAN PATENT APPLICATION

(11) **EP 2 281 535 A1**
(43) Date of publication of application: **09.02.2011**
(21) Application number: 09754645.1
(22) Date of filing: 25.05.2009
(51) Int. Cl.: A61F 13/15, A61F 13/534, A61F 13/539, A61F 13/56

(54) **ABSORPTIVE ARTICLE AND SANITARY NAPKIN**

(30) Priority: 28.05.2008 JP 2008140054
(71) Applicant: Unicharm Corporation, Ehime 799-0111 (JP)
(72) Inventor: The designation of the inventor has not yet been filed
(74) Representative: Eke, Philippa Dianne
(86) International application number: PCT/JP2009/059515
(87) International publication number: WO 2009/145137

(57) **Abstract**

An absorbent article (100) comprises a liquid-permeable top sheet (10), a liquid-impermeable back sheet (40), and an absorber (30) mounted between the top sheet (10) and the back sheet (40). A coating region (400) to which an adhesive (60) is applied is formed in a flat surface direction, on a surface (42) of the back sheet (40) which faces clothing. An endothermic material (50) is provided inside the absorber (30). A region (410) which, with respect to the thickness direction of the absorbent article (100), does not face an arrangement region (225) in which the heat endothermic material (50) is provided is formed in at least a part of the coating region (400) to which the adhesive (60) is applied.

## Description

### BACKGROUND OF THE INVENTION

### 1. Field of the Invention

The present invention relates to an absorbent article and a sanitary napkin each including a liquid permeable top sheet, a liquid impermeable back sheet, and an absorber interposed between the top sheet and the back sheet.

### 2. Description of the Related Art

Conventionally, in order to absorb a body fluid such as menstrual blood excreted from a wearer, absorbent articles such as sanitary napkins have been widely used (for example, see Patent document 1).

Generally, such an absorbent article has a liquid permeable top sheet, a liquid impermeable back sheet, and an absorber interposed between the top sheet and the back sheet. In addition, when a wearer puts the absorbent article on, the top sheet comes into contact with a skin surface of the wearer, and the back sheet comes into contact with an underwear surface.

Moreover, an adhesive is provided on a surface of the back sheet that comes into contact with the underwear surface. The adhesive adheres to the underwear surface to thereby prevent the absorbent article from being displaced or twisting on the underwear.

### [Citation List]

### [Patent Literature]

Patent document 1: Japanese Patent Application Publication No.2001-190596
Patent document 2: Japanese Patent No.3922722

### SUMMARY OF THE INVENTION

Body fluid such as menstrual blood excreted from a wearer begins transpiring immediately after excretion to an absorbent article. For this reason, temperature and humidity in a space between a skin surface of the wearer and the absorbent article rise, thereby leading to a problem that the wearer feels stuffiness.

Here, JP Pat No. 3922722 has disclosed an absorbent article including an endothermic material which causes endothermic reaction with an attachment of a body fluid. In principle, such absorbent article can suppress increases in temperature and humidity when the wearer excretes the body fluid such as menstrual blood. The absorbent article, however, is intended as a toilet training diaper for children, and is for causing a wearer to recognize excretion of body fluid in a way that the absorbent article forcefully turns back body fluid having a temperature lowered by the endothermic material and thereby attaches the body fluid to a skin surface of the wearer. Therefore, the absorbent article is not intended to suppress the stuffiness when the body fluid such as menstrual blood is excreted.

Further, in the absorbent article, the adhesive provided on the back sheet has very high temperature dependency. Therefore, a temperature of the adhesive may also be reduced by the body fluid having its temperature reduced by the endothermic material, and the adhesive may harden. Accordingly, when the endothermic material is disposed in a sanitary napkin, for example, a reduction in the adhesive strength of the adhesive may allow the absorbent article from being displaced or twisting on the underwear.

Thus, the present invention has been made in consideration of the above-mentioned tasks. An object of the present invention is to provide an absorbent article and a sanitary napkin that suppress stuffiness felt by a wearer when a body fluid such as menstrual blood is excreted, the absorbent article being also less likely to be displaced or twist on underwear while being worn.

A first aspect of the present invention is summarized by an absorbent article including a liquid permeable top sheet, a liquid impermeable back sheet, and an absorber interposed between the top sheet and the back sheet, wherein a coating region of an adhesive is formed in a planar direction on a surface of the back sheet facing clothing, an endothermic material is disposed inside the absorber, and a region not facing an arrangement region of the endothermic material in a thickness direction of the absorbent article, is formed at least in a part of the coating region of the adhesive.

In the first aspect, the endothermic material may be disposed inside the absorber on a top sheet side.

In the first aspect, a hydrophobic sheet may be disposed between the back sheet and the absorber.

A second aspect of the present invention is summarized by a sanitary napkin including a liquid permeable top sheet, a liquid impermeable back sheet, and an absorber interposed between the top sheet and the back sheet, wherein a coating region of an adhesive may be formed in a planar direction on a surface of the back sheet facing clothing, an endothermic material may be disposed inside the absorber, and a region not facing an arrangement region of the endothermic material in a thickness direction of the absorbent article may be formed in at least a part of the coating region of the adhesive.

In the second aspect, the sanitary napkin may further include a wing extending in a width direction, wherein in the sanitary napkin, a front region, a central region, and a rear region are provided continuously in a longitudinal direction of the absorber, the endothermic material is disposed inside the absorber in an arrangement region including at least a central region of the absorbent article, and a region, in a longitudinal direction, of the central region is a region, in a longitudinal direction, of the wing.

In the second aspect, in the sanitary napkin, an embossed groove may be formed in both side portions in a width direction of the absorber, the embossed groove is formed along a longitudinal direction of the sanitary napkin, and the endothermic material may be interposed between the embossed grooves.

As described above, according to the present invention, it is possible to provide an absorbent article and a sanitary napkin capable of suppressing stuffiness that the wearer feels when a body fluid such as menstrual blood or urine is excreted, the absorbent article being also less likely to be displaced or twist on underwear while being worn.

### BRIEF DESCRIPTION OF THE DRAWINGS

[Fig. 1] Fig. 1 is a plan view of an absorbent article according to a first embodiment of the present invention.
[Fig. 2] Fig. 2 is a sectional view of the absorbent article according to the first embodiment of the present invention.
[Fig. 3] Fig. 3 is a diagram showing an arrangement region of an endothermic material and a coating region of an adhesive of the absorbent article according to the first embodiment of the present invention.
[Fig. 4] Fig. 4 is a plan view of an absorbent article according to a second embodiment of the present invention.
[Fig. 5] Fig. 5 is a sectional view of the absorbent article according to the second embodiment of the present invention.
[Fig. 6] Fig. 6 is a sectional view of an absorbent article according to a third embodiment of the present invention.

### DETAILED DESCRIPTION OF THE PREFERRED EMBODIMENTS

### (Absorbent Article According to First Embodiment of the Present Invention)

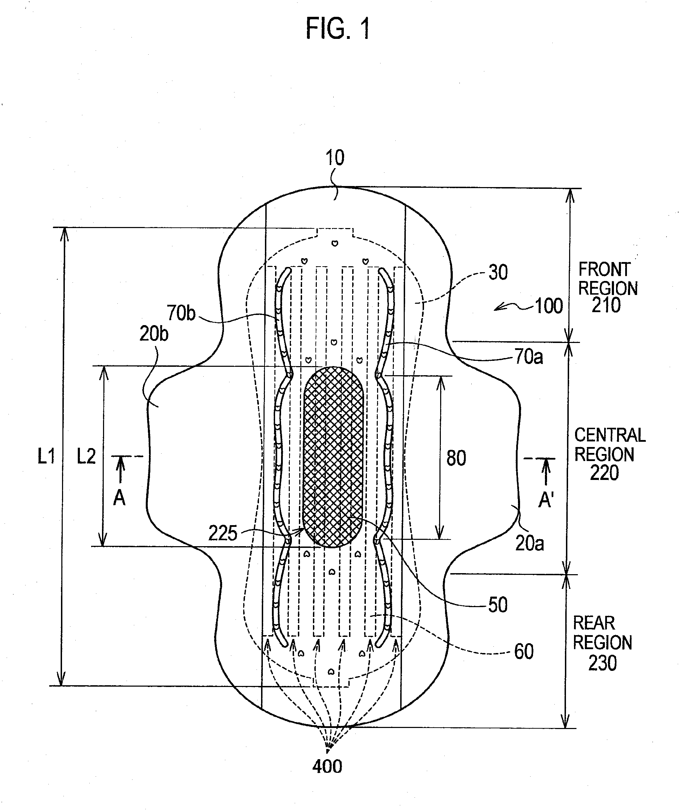

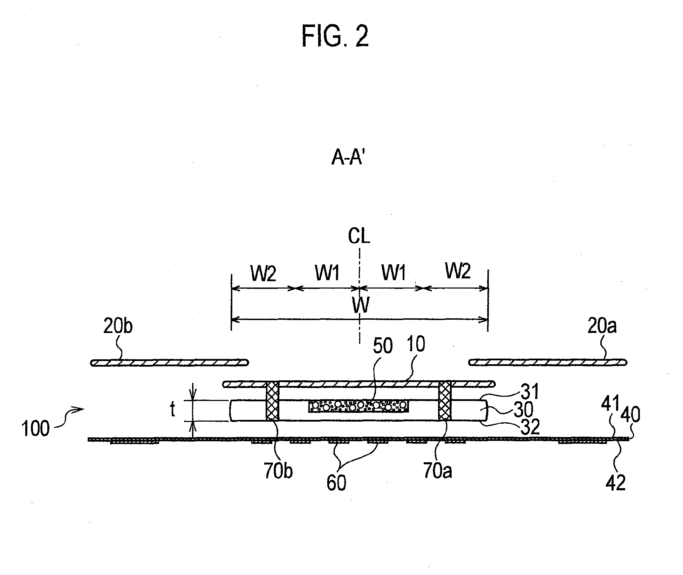

With reference to Figs. 1 to 2, a description will be given of an absorbent article according to a first embodiment of the present invention. Fig. 1 is a plan view of an absorbent article 100 according to the first embodiment of the present invention, and Fig. 2 is a sectional view of the absorbent article 100 taken along an A-A' line.

The absorbent article 100 is used as a sanitary napkin, for example. For storing in an individual packaging container and the like, the absorbent article 100 is folded inward in three or four, and is enclosed in the container. The whole shape of the absorbent article 100 may be rectangular, elliptical, gourd-shaped, etc., and is not particularly limited as long as the shape suits shapes of the wearer's body and underwear. Moreover, as an external dimension of the absorbent article 100, a dimension in a longitudinal direction is preferably in a range of "100 to 500 mm", and specifically, a range of "150 to 350 mm" is more preferable. Further, a dimension in a width direction is preferably in a range of "30 to 200 mm", and specifically, a range of "40 to 180 mm" is more preferable.

As shown in Fig. 1, in the present embodiment, the absorbent article 100 has a front region 210, a central region 220, and a rear region 230 provided continuously in a longitudinal direction (planar direction) of an absorber 30. The front region 210 is a region contacting a skin surface on a belly side of the wearer, the central region 220 is a region contacting a skin surface of a crotch part of the wearer, and the rear region 230 is a region contacting a skin surface on a buttocks side of the wearer.

Here, the central region 220 is a region to which a largest amount of a body fluid such as menstrual blood attaches when the wearer puts the absorbent article 100 on. For example, a region near the center in the width direction and in the longitudinal direction of the absorbent article 100 may be defined as the central region 220. Furthermore, when embossed grooves 70a to 70b (described later) contoured in a crotch shape are formed in the absorbent article 100, a crotch shape portion 80 of the embossed grooves 70a to 70b (described later) may be defined as the central region 220 as shown in Fig. 1.

Moreover, in the absorbent article 100 according to the present embodiment, wings 20a to 20b extending in the width direction of the absorbent article 100 are formed so as to fix the absorbent article 100 to the underwear. When the wings 20a to 20b are thus formed, a region corresponding to the wings 20a to 20b may be defined as the central region 220. Specifically, a region in the longitudinal direction in which the wings 20a to 20b are provided may be defined as a region extending in a longitudinal direction of the central region 220. In the present embodiment, a description will be given based on an assumption that the region in the longitudinal direction in which the wings 20a to 20b are provided is the region in the longitudinal direction of the central region 220.

As shown in Fig. 2, the absorbent article 100 has a liquid permeable top sheet 10, a liquid impermeable back sheet 40, and an absorber 30 interposed between the top sheet 10 and the back sheet 40. The top sheet 10 comes in contact with the skin surface of the wearer, and the back sheet 40 comes in contact with an underwear surface. In the absorbent article 100, it is preferable that the top sheet 10, the absorber 30, and the back sheet 40 be joined in order to prevent separation between layers. Specifically, in the absorbent article 100, it is preferable that the top sheet 10 and the back sheet 40 be joined to each other in a periphery portion of the absorber 30, so that the absorber 30 is enclosed therein. As a method for joining the top sheet 10 and the back sheet 40, it is possible to employ any processing of heat embossing, ultrasonic wave, or a hot melt adhesive or a combination of these.

As shown in Figs. 1 to 2, in the absorbent article 100, the embossed grooves 70a to 70b (the so-called hinge) are formed in the top sheet 10 and the absorber 30 in order to join the top sheet 10 and the absorber 30. The embossed grooves 70a to 70b are formed in a pattern (embossed pattern) contoured a shape of the crotch part so that the absorbent article 100 more fits the crotch part.

As shown in Figs. 1 to 2, the embossed grooves 70a to 70b are formed so as to extend in a longitudinal direction in both side portions in the width direction of the absorber 30. Here, in the present embodiment, when an interval in the width direction of the absorber 30 is W as shown in Fig. 2, each of the both side portions in the width direction of the absorber 30 is defined as a region shown as an interval W2 exceeding an interval W1 extending outward from the center of the absorber 30 in the width direction. For example, when the interval W1 is "10 mm", a region shown as the interval W2 exceeding "10 mm" extending outward from the center of the absorber 30 in the width direction is defined as each of the both side portions. Note that the both side portions are not limited to the content mentioned above. For example, a ratio of the interval W1 to the interval W2 is set to "1:1" or "2:1", and the both side portions each may be defined as a region of an interval W2 corresponding to this ratio. In addition, an interval in the width direction between the embossed groove 70a and the embossed groove 70b is preferably "20 to 60 mm".

Moreover, in the absorbent article 100, in order to prevent side leakage of the body fluid such as menstrual blood, gathers (not shown) including an elastic material such as a resilient material may be provided in the both side portions in the width direction of the absorber 30.

The top sheet 10 employs a nonwoven fabric as a base material. Note that the base material for the top sheet 10 is not particularly limited to a specific one, but any material can be used as long as the material is a sheet-like material having a liquid permeable structure such as a woven fabric or a perforated plastic sheet. Both natural fibers and chemical fibers can be used as a material for the woven fabrics or nonwoven fabrics. Examples of the natural fibers include cellulose such as crushed pulp and cotton. Examples of the chemical fibers include regenerated cellulose such as rayon and fibril rayon, semisynthetic cellulose such as acetate and triacetate, thermoplastic hydrophobic chemical fibers, and thermoplastic hydrophobic chemical fibers subjected to hydrophilization treatment. Examples of the thermoplastic hydrophobic chemical fibers include single fibers such as polyethylene (PE), polypropylene (PP), and polyethylene terephthalate (PET), fibers obtained by graft polymerization of polyethylene and polypropylene, and composite fibers of a sheath-core structure and the like.

In particular, as the method of web forming of nonwoven fabrics, any one of dry methods (the carding method, the spun bonding method, the melt-blowing method, the air-laid method, etc.) and wet methods or a combination of the above-mentioned methods may be used. Moreover, the method of bonding may be thermal bonding, needle punching, and chemical bonding, but is not particularly limited to these methods. Alternatively, spunlace formed in a sheet form by the hydroentangling method may be used as a nonwoven fabric.

The back sheet 40 has an adhesive 60 adhering to the underwear of the wearer for preventing displacement disposed on a surface on the underwear side. A peeling sheet (not shown) is disposed by adhesion on an external surface of the adhesive 60. A material which can be employed for the back sheet 40 includes, films mainly formed of polyethylene, polypropylene and the like, air permeable resin films, a combination of air permeable resin films joined to a nonwoven fabric such as spun bond, spunlace and the like, plural layers of SMS, and others. Taking account flexibility of a level which does not reduce the fittingness, it is preferable that the back sheet 40 employ a film mainly formed of a low density polyethylene (LDPE) resin and having a basis weight within "15 to 30 g/m^{2"}, for example.

The absorber 30 may be a product formed of a hydrophilic fiber or a polymer covered with a coating material, or may be an air-laid sheet formed into a sheet form by an air laid method.

Examples of the hydrophilic fibers include cellulose such as crushed pulp and cotton, regenerated cellulose such as rayon and fibril rayon, semisynthetic cellulose such as acetate and triacetate, granular polymers, fibrous polymers, thermoplastic hydrophobic chemical fibers, and thermoplastic hydrophobic chemical fibers subjected to hydrophilization treatment. These can be used alone or being combined. Out of these, in consideration of lower costs and workability of forming the absorber, it is preferable that crushed pulp be used.

As an example of the polymers, granular polymers such as sodium acrylate copolymer having absorbency and hygroscopicity are generally used. Alternatively, in order to obtain other properties, a granular deodorant material such as silver, copper, zinc, silica, activated carbon, aluminosilicate compounds, and zeolite may be used.

For the coating material, for example, a woven fabric, a nonwoven fabric, or the like of any type without particular limitation is usable as long as the fabric is liquid permeable and has barrier properties sufficient to prohibit a high polymer absorbent and an endothermic material (described later) from passing through the fabric. Both natural fibers and chemical fibers are usable as a material for the woven fabric and the nonwoven fabric. Examples of the natural fibers include cellulose such as crushed pulp and cotton. Examples of the chemical fibers include regenerated cellulose such as rayon and fibril rayon, semisynthetic cellulose such as acetate and triacetate, thermoplastic hydrophobic chemical fibers, and thermoplastic hydrophobic chemical fibers subjected to hydrophilization treatment.

Moreover, in consideration of lower cost and higher barrier properties, it is preferable to use as the nonwoven fabric a tissue mainly composed of crushed pulp and formed by the wet method.

When an air-laid sheet is used as the absorber 30, it is preferable that the thickness be 0.3 to 5.0 mm. Examples of the air-laid sheet include an air-laid sheet formed of fibers and a granular polymer in a sheet form by use of a binder and the like. In the air-laid sheet, the granular polymer may be distributed as a layer, or may be concentrated in a thickness direction.

Moreover, the absorber 30 may have embossing formed therein in order to prevent deformation and twist during being worn, or in order to adjust a thickness. The absorber 30 can be embossed by passing between a patterned embossing roll and a flat roll. While either a lattice pattern, a dot pattern, and a wave pattern is employed as a pattern of the embossing roll, a lattice pattern is preferable because of its thickness adjustability.

A dimension in the longitudinal direction of the absorber 30 is preferably in a range of "90 to 490 mm", and specifically, it is more preferable in a range of "140 to 340 mm". Moreover, a dimension in the width direction is preferably in a range of "25 to 100 mm", and specifically, it is more preferable in a range of "35 to 80 mm".

As shown in Fig. 2, an endothermic material 50 is disposed in the absorber 30. When coming into contact with fluids such as body fluids, the endothermic material 50 is water-soluble and causes endothermic reaction to absorb surrounding heat energy. Examples of the endothermic material 50 include water-soluble materials that cause the endothermic reaction by hydration, such as potassium chloride, sodium chloride, sodium acetate, potassium nitrate, urea, sodium bicarbonate, xylitol, and trehalose. Out of these, from a viewpoint of irritativeness to the skin at the time of dissolution and stability such as long term storage, potassium chloride is preferable.

### (Arrangement Area of Endothermic Material)

A description will be given of an arrangement region of the endothermic material 50 in the absorbent article 100 according to the present embodiment. For an efficient generation of the endothermic reaction with excreted menstrual blood, it is preferable that an arrangement region 225 of the endothermic material 50 include at least a region contacting the crotch part of the wearer.

In the present embodiment, the endothermic material 50 is disposed in an arrangement region including at least a central region 220 in the longitudinal direction (planar direction) of the absorbent article 100, and is disposed inside the absorber 30 in the thickness direction of the absorbent article 100. A region in a longitudinal direction of the central region 220 corresponds to a region in the longitudinal direction of the wings 20a to 20b. Moreover, the endothermic material 50 is disposed in a region between the embossed groove 70a and the embossed groove 70b in the width direction (planar direction) of the absorbent article 100.

In Fig. 1, the arrangement region 225 in the planar direction of the endothermic material 50 is indicated by a mesh pattern. Here, as shown in Fig. 1, the arrangement region including at least the central region 220 may be a region within the central region 220, or may be a region including a part of the central region 220 and an region outside of the central region 220. As shown in Fig. 1, when a length in the longitudinal direction of the absorber 30 is defined as "L1", it is preferable that a length in the longitudinal direction of the arrangement region 225 be a length "L2" having a 30 to 50% of "L1" or more.

Here, assume if the length in the longitudinal direction of the arrangement region 225 is less than 30% of the length in longitudinal direction of the absorbent article 100. In that case, when an amount of menstrual blood is less, or when the wearer does not wear the absorbent article 100 on an appropriate position, the endothermic material 50 may have difficulty in causing the endothermic reaction.

On the other hand, when the length in the longitudinal direction of the arrangement region 225 is not less than 50%, the endothermic material 50 may cause the endothermic reaction with sweat or the like excreted from the crotch part of the wearer other than menstrual blood. Accordingly, chilliness may be given to the wearer more than needed.

Therefore, it is preferable that the length in the longitudinal direction of the arrangement region 225 be 30 to 50% of the length in the longitudinal direction of the absorbent article 100.

More preferably, the arrangement region 225 of the endothermic material 50 includes not only the central region 220 but also the center in the longitudinal direction of the absorbent article 100. The center of the absorbent article 100 may be the center of the entire absorbent article 100, or may be a point on the center line that divides the wings 20a and 20b equally in the longitudinal direction. When the both side portions of the absorber 30 are formed to be curved in a form projected inward in the width direction, the center in the longitudinal direction of the absorbent article 100 may be a narrowest portion in the width of the absorber 30.

Moreover, when a middle higher part projecting in the thickness direction is formed in the absorbent article 100, the center of the absorbent article 100 may be the center in a planar direction of the middle higher part. The middle higher part may be formed by laminating multiple absorbers 30 having different sizes, or may be formed so as to have its basis weight of the absorber 30 larger than basis weights in other regions.

Moreover, a density of the endothermic material 50 in the planar direction of the arrangement region 225 may be uniform, or may be formed so as to be high in the central region 220.

It is preferable that as for a position in the thickness direction, the endothermic material 50 is disposed in a position where uncomfortable feelings to the wearer such as chilliness by the endothermic reaction of the endothermic material 50 can be avoided. The endothermic material 50 may be disposed, for example, between the top sheet 10 and the absorber 30 or inside the top sheet. However, it is preferable that the endothermic material 50 be placed inside the absorber 30 in order not to repeat the endothermic reactions in a long period of time or not to give chilliness.

In the present embodiment, the endothermic material 50 is disposed inside the absorber 30 on the top sheet 40 side. Specifically, in the present embodiment, in the thickness direction of the absorber 30, a density of the endothermic material 50 on the top sheet 10 side is higher than a density of the endothermic material 50 on the back sheet 40 side. In other words, the endothermic material 50 inside the absorber 30 has different densities in the thickness direction of the absorber 30.

For example, as shown in Fig. 2, an interval in the thickness direction of the absorber 30 is defined as "t." Moreover, a half of the interval "t" is defined as "t1." In the present embodiment, in the thickness direction, the density of the endothermic material 50 inside the absorber 30 in the interval "t1" extending from a surface 31 of the absorber 30 on the top sheet side is higher than the density of the endothermic material 50 inside the absorber 30 in the interval "t1" extending from a surface 32 of the absorber 30 on the back sheet side.

In the absorbent article 100, when an interval (the first interval) between the surface 31 on the top sheet 10 side of the absorber 30 and the endothermic material 50 and an interval (the second interval) between the surface 32 on the back sheet 40 side of the absorber 30 and the endothermic material 50 are provided in the thickness direction of the absorber 30, the interval between the surface 32 and the endothermic material 50 may be formed so as to be larger than the interval between the surface 31 and the endothermic material 50.

### (Grain Diameter of Endothermic Material)

A description will be given of a grain diameter of the endothermic material 50 according to the present embodiment. Endothermic materials having different grain diameters are disposed in the endothermic material 50 according to the present embodiment. Specifically, the endothermic material 50 includes endothermic materials having a grain diameter in a range of 150 µm to 850 µm and having different grain diameters. The grain diameter of the endothermic material is not particularly limited, but may be within a range of 300 to 800 µm or within a range of 350 to 600 µm from a viewpoint of feeling of foreign objects and a viewpoint of productivity.

Here, when components of the endothermic materials are the same, the endothermic material having a larger grain diameter takes more time until completely dissolves. This is because the endothermic material begins to dissolve from an uppermost surface of the grains when dissolving by hydration, and therefore, the grain having larger diameter, that is, having larger volume takes more time to completely dissolve.

In order to cause the endothermic reaction even in the case of long-time excretion or repeated excretion, a larger grain diameter is more preferable. However, if the absorber 30 includes only an endothermic material having a larger grain diameter, rigidity of the absorber 30 increases, and therefore, feeling of foreign matters is given to the wearer.

Accordingly, in order to cause the endothermic material 50 to cause the endothermic reaction for a long time and to prevent an excessive increase in the rigidity of the absorber 30, it is preferable that endothermic materials having different grain diameters be included.

### (Configuration of Endothermic Material in Absorber)

A description will be given of a configuration of the endothermic material 50 according to the present embodiment. As for the endothermic material 50, a sheet covering only the endothermic material 50 with a coating material may be disposed on an upper surface of the absorber 30, or the endothermic material 50 may be disposed as a layer between pulps. Further, the endothermic material 50 may be sandwiched between the top sheet 10 and the absorber 30.

Here, since the endothermic material 50 is water-soluble , for example, when a region of the endothermic material 50 where a diameter of a grain is small dissolves at a first excretion of menstrual blood, a space is generated in the endothermic material 50. In this case, when the menstrual blood is repeatedly excreted, it becomes difficult for the menstrual blood to move from the top sheet 10 to the back sheet 40. As a result, the menstrual blood stagnates in the top sheet 10, and thus the wearer may feels uncomfortable.

As mentioned above, it is preferable that the endothermic material 50 be blended with pulp and the like, and be dispersed to some extent. Specifically, the absorber 30 is preferably obtained by mixing pulp in a rage of 60 to 98%, the granular endothermic material 50 in a range of 40 to 2%, and a granular polymer in a range of 20 to 0%, the mixture is then covered with a tissue, and subsequently the mixture is formed into a sheet having a basis weight of 100 to 2000 g/m² and a height of 1 to 50 mm by embossing. Embossing is performed for preventing deformation of the absorber, and an embossed area rate is preferably in a range of 10 to 100%, and specifically, in a range of 30 to 80%.

It is preferable that the endothermic material 50 in the absorber 30 has a configuration which allows the endothermic material 50 to repeat the endothermic reactions by the body fluid such as menstrual blood in a long period of time, or a configuration that gives no chilliness. Specifically, for an upper layer material (top sheet 10 side), pulp of 50 to 150 gsm and potassium chloride of 0.5 to 3.0 g are blended. For a lower layer material (back sheet 40 side), pulp of 50 to 200 gsm and a high polymer absorbent of 0.1 to 0.5 g are blended. Then, the upper layer material and the lower layer material are layered, covered with a tissue, and subsequently, embossed. This may be used as the endothermic material 50.

### (Mixing Amount of Endothermic Material)

A description will be given of a mixing amount of the endothermic material 50 in the absorber 30 according to the present embodiment. The mixing amount of the endothermic material 50 is influenced by the variety of heat of dissolution and solubility, which depend on a kind of the endothermic material 50, and also by an arrangement position of the endothermic material 50. Here, in consideration of suppressing sharp increases in temperature and humidity in the space between the skin surface and the absorbent article 100 and of giving no uncomfortable feelings to the wearer such as chilliness, a weight of the endothermic material 50 per sheet in the absorbent article 100 is preferably in a range of 0.1 to 10 g, and more specifically, in a range of 0.5 to 5 g.

As an example, a description will be given of a case where the absorbent article 100 is used as a sanitary napkin and potassium chloride is used as the endothermic material 50.

It is known that an average absorbed amount per a napkin in a day (the first to third day) during a menstrual period when much menstrual blood is excreted is generally approximately 6.0 ml. It is also known that solubility of potassium chloride is approximately 27.0% in an experiment liquid (physiological saline) adjusted to 37°C ± 5°C.

From these, potassium chloride that can dissolve to 6.0 ml of menstrual blood is approximately 2.0 g, and even when not less than potassium chloride 2.0g is mixed, the excessive potassium chloride does not cause the endothermic reaction. Accordingly, when potassium chloride is used in sanitary napkins for days with much menstrual blood loss, a mixing amount of potassium chloride is preferably in a range of 0.5 to 3.0 g, and more preferably in a range 1.0 to 2.0 g.

### (Disposition of Adhesive and Endothermic Material)

In the absorbent article 100 according to the present embodiment, a description will be given of disposition of the adhesive 60 and the endothermic material 50.

In the absorbent article 100 according to the present embodiment, coating regions of an adhesive are formed in a planar direction on a surface of the back sheet 40 side facing clothing.

Specifically, as shown in Fig. 2, the adhesive 60 is disposed in the back sheet 40. Here, the surface of the back sheet 40 facing underwear (clothing) is a side of the back sheet 40 opposite to a side facing the absorber 30 in the thickness direction of the absorbent article 100. In other words, as shown in Fig. 2, the surface of the back sheet 40 facing the underwear is a surface 42 of the back sheet 40 on the side opposite to the absorber 30. The surface of the back sheet 40 facing the absorber 30 is a surface 41 of the absorber 30 side.

Moreover, as shown in Fig. 1, the coating regions 400 of the adhesive 60 are formed in the planar direction of the absorbent article 100. Specifically, in the example of Fig. 1, the coating regions 400 of the adhesive 60 are applied in a longitudinal direction of the absorbent article with a width dimension of 6 mm and at an interval of 5 mm to be in six strips each having a blind shape. In the example of Fig. 1, preferably, a longitudinal dimension of the coating regions 400 of the adhesive 60 is longer than at least a dimension in the longitudinal direction of the endothermic material 50. Preferably, a basis weight of the adhesive 60 is 10 to 200 g/m². By coater coating, bead coating, etc., the adhesive 60 can be disposed (coated) in the back sheet 40 in a uniform pattern, a striped pattern, or a dotted pattern.

Moreover, in the absorbent article 100 according to the present embodiment, a region 410 not facing the arrangement region 225 of the endothermic material 50 in the thickness direction of the absorbent article 100 is formed at least in a part of the coating regions 400 of the adhesive 60.

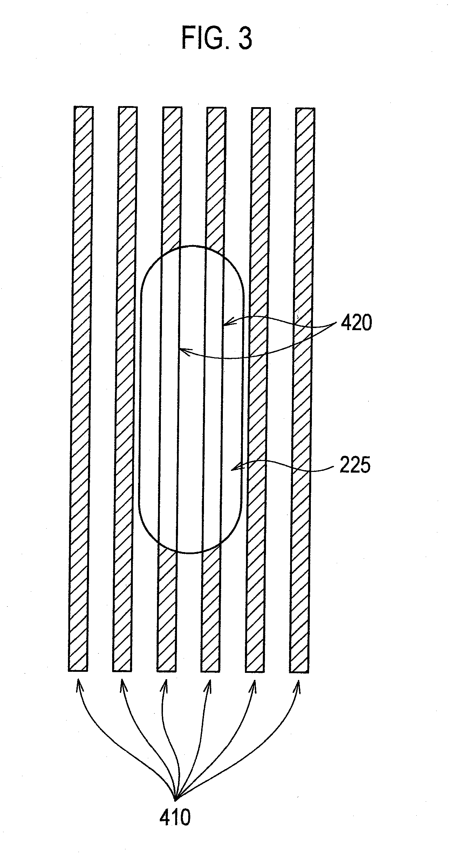

Specifically, with reference to Fig. 3, a description will be given. Fig. 3 shows a plan view of the coating regions 400 of the adhesive 60 and the endothermic material 50 in Fig. 1. As shown in Fig. 3, when the absorbent article 100 is seen in the thickness direction of the absorbent article 100 from above, the coating regions 410 that do not overlap (not overlay) the arrangement region 225 of the endothermic material 50 are formed in a part of the coating regions 400 of the adhesive 60. Moreover, coating regions 420 that overlap the arrangement region 225 of the endothermic material 50 are formed.

In other words, the coating regions 400 of the adhesive 60 that do not overlap (not overlay) the arrangement region 225 of the endothermic material 50 when the absorbent article 100 is seen in the thickness direction of the absorbent article 100 from above are the coating regions 410 not facing the arrangement region 225. Moreover, the coating regions 400 of the adhesive 60 that overlap (overlay) the arrangement region 225 of the endothermic material 50 are the coating regions 420 facing the arrangement region 225.

A region obtained by joining the coating regions 410 not facing the arrangement region 225 of the endothermic material 50 and the coating regions 420 facing the arrangement region 225 of the endothermic material 50 is the entire coating regions 400 of the adhesive 60.

### (Material of Adhesive)

Preferably, the adhesive 60 has a tack property at room temperature. Accordingly, examples of the adhesive 60 include pressure sensitive adhesives. Moreover, examples of a base polymer for the adhesive 60 include rubber-like polymers such as SEBS, SBS, and SIS, olefin polymers such as straight-chain low density polyethylene, polyvinyl alcohol, carboxyl methylcellulose, gelatin composed of water soluble polymers, etc. and polyvinyl acetate, sodium polyacrylate, etc. composed of water swelling polymers.

Moreover, a configuration of the adhesive 60 includes a configuration including a base polymer, a tackifier, an oil, and a stabilizer. Preferably, the adhesive 60 according to the present embodiment has lower dependency on temperature. In other words, preferably, the adhesive strength cannot easily deteriorate even when the temperature of the body fluid is reduced by the endothermic material 50. Moreover, the adhesive 60 itself may have softness to thereby maintain the adhesive strength at a low temperature. For example, an oil ratio of the adhesive 60 may be increased, or a base polymer with a lower molecular weight may be used. Specifically, an adhesive obtained by adding 15 to 25% of SEBS, 40 to 70% of a tackifier, 15 to 35% of an oil, and 0.1 to 1.0% of a stabilizer such as antioxidants or anti-fluorescent agents is given as an example.

According to the absorbent article 100 of the first embodiment of the present invention, the endothermic material 50 is disposed inside the absorber 30. Accordingly, according to the absorbent article 100, increases in temperature and humidity of the absorber 30 by the endothermic reaction of the endothermic material 50 when the body fluid such as menstrual blood is excreted is suppressed. In other words, according to the absorbent article 100, it is possible to suppress the stuffiness that the wearer feels when the body fluid such as menstrual blood or urine is excreted.

Further, when the water-soluble material used as the endothermic material 50 is dissolved into the blood, the solute concentration in the blood plasma increases. Therefore, the osmotic pressure of the blood plasma increases, and the water included in the red blood cell is discharged. This results in a contraction of the red blood cell. Thus, the volume of the red blood cell is decreasesd. When the volume of the red blood cell is decreased in this manner, surface tension of the blood is increasesd, thereby contact angle of the blood with respect to the top sheet 10 is increasesd. In other words, wettability leakage probability of the red blood cell is decreasesd.

As described above, when the water-soluble material is dissolved into the blood that has once passed through the top sheet 10, the leakage probability wettability of the blood is decreasesd. Therefore, the blood is not easily transmitted returnedback through the top sheet 10 to the skin surface through the top sheet 10side. Thus, the wearer can feel sense of dryness.

According to the absorbent article 100 of the first embodiment of the present invention, the coating regions 410 not facing the arrangement region 225 of the endothermic material in the thickness direction of absorbent article 100 are formed at least in a part of the coating regions 400 of the adhesive 60. Accordingly, even when the temperature of the body fluid included in the absorber 30 is reduced by the endothermic material 50, there is less influence upon reduction in temperature of the adhesive 60 in the coating regions 410.

Here, generally, since the adhesive 60 has high temperature dependency and hardens due to lowering of temperature, the adhesive 60 may deteriorate the adhesive strength. The deterioration of the adhesive strength of the adhesive 60 causes problems that the absorbent article attached to the underwear is displaced or twists thereby to give the wearer feeling of foreign objects and to allow leakage of the body fluid such as menstrual blood.

According to the absorbent article 100 of the present embodiment, even when the adhesive 60 is provided which is highly dependent on temperature and deteriorates the adhesive strength due to reduction in temperature, reduction in temperature of the adhesive 60 can be reduced. Accordingly, the absorbent article can be prevented from being displaced or twisting.

Thus, according to the absorbent article 100 of the first embodiment of the present invention, it is possible to suppress the stuffiness that the wearer feels when the body fluid such as menstrual blood is excreted, and to reduce occurrence of a case where the absorbent article 100 is displaced or twisted on the underwear due to reduction in the adhesive strength of the adhesive 60.

Moreover, according to the absorbent article 100 of the first embodiment of the present invention, the endothermic material 50 is disposed inside the absorber 30 on the top sheet 10 side. Accordingly, even when a small amount of the body fluid such as menstrual blood is excreted, the endothermic reaction can be generated more securely in the endothermic material 50 thereby suppressing increases in temperature and humidity.

Moreover, according to absorbent article 100 of the first embodiment of the present invention, the endothermic material 50 is disposed in the region including at least the central region 220 of the absorbent article 100. Additionally, in the present embodiment, the central region 220 corresponds to the region of the wings 20a to 20b in the longitudinal direction.

Here, the absorbent article 100 is generally used so that a region between the wing 20a and wing 20b may come in contact with the crotch part when the absorbent article 100 is put on. Accordingly, by defining the region of the wings 20a to 20b in the longitudinal direction as the central region 220 and disposing the endothermic material 50 in the region including at least the central region 220, the body fluid from the wearer such as menstrual blood can be made to attach to the endothermic material 50 more securely so as to cause the endothermic reaction.

Moreover, in the absorbent article 100 according to the first embodiment of the present invention, the embossed grooves 70a to 70b are formed in order to join the top sheet 10 and the absorber 30. The endothermic material 50 is disposed in the region between the embossed groove 70a and the embossed groove 70b.

Accordingly, in the absorbent article 100 according to the first embodiment of the present invention, the top sheet 10 and the absorber 30 are formed so as not to easily separate from each other. For this reason, the body fluid from the wearer such as menstrual blood can be made to attach to the endothermic material 50 more securely to cause the endothermic reaction. Further, according to the absorbent article 100, the endothermic material 50 is disposed in the region between the embossed groove 70a and the embossed groove 70b. Accordingly, even when the absorbent article 100 is twisted, endothermic material 50 is less likely to come out of the absorber 30.

### (Absorbent Article According to Second Embodiment of the Present Invention)

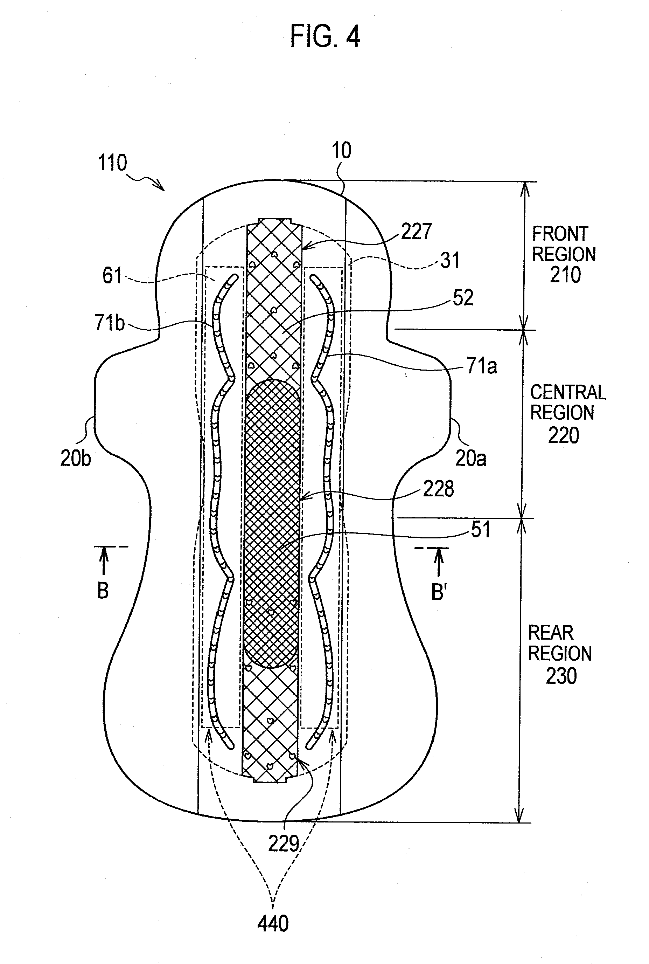

With reference to Figs. 4 to 5, a description will be given of an absorbent article according to a second embodiment of the present invention focusing on differences from the absorbent article according to the first embodiment of the present invention.

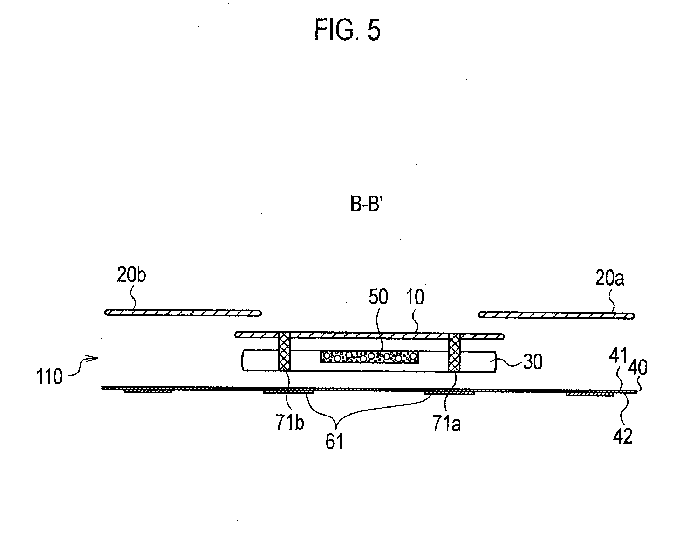

Fig. 4 is a plan view of the absorbent article 110 according to the second embodiment of the present invention, and Fig. 5 is a sectional view of the absorbent article 110 taken along a B-B' line.

The absorbent article 110 according to present embodiment has a longer rear region 230 in the longitudinal direction than the absorbent article 100 according to the first embodiment. In other words, the absorbent article 110 has a longer region contacting the skin surface on the buttocks side (tailbone side) of the wearer. The absorbent article 110 is configured so as to absorb menstrual blood, etc. that flows along the body of the wearer (for example, buttocks), for example, even when the wearer is lying (for example, lying on her back).

Additionally in the absorbent article 110, the embossed grooves 71a to 71b are formed in the top sheet 10 and the absorber 30 to be contoured in a shape of the crotch part so that the absorbent article 110 more fits the crotch part.

Here, as shown in Fig. 4, an endothermic material 51 is disposed in an arrangement region 228, which is a central portion in a longitudinal direction of the absorbent article 110. As shown in Fig. 4, an endothermic material 52 is disposed in arrangement region 227 and an arrangement region 229, which are both end portions in the longitudinal direction of the absorbent article 110. The both end portions in the longitudinal direction of the absorbent article 110 mean a belly region (arrangement region 227) and a buttocks region (arrangement region 229) with respect to the region of the central portion mentioned above when the wearer puts the absorbent article 110 on.

Meanwhile, a mixing percentage of the endothermic material 52 in the arrangement region 227 and arrangement region 229 of the endothermic material 52 is lower than a mixing percentage of the endothermic material 51 in the arrangement region 228. The mixing percentage denotes a mixing amount per unit area, for example, a weight of the endothermic material per unit area. Endothermic materials having different particle diameters may be disposed in the endothermic materials 51 to 52.

Further, in the absorbent article 110 according to the second embodiment of the present invention, leakage probability of the red blood cell can decreased since the water-soluble material is dissolved into the blood that has once passed through the top sheet 10. Therefore, the blood is not easily returned to the skin surface through the top sheet 10. Thus, the wearer can feel sense of dryness.

Moreover, as shown in Fig. 4, coating regions 440 of an adhesive 61 are formed in a planar direction of the absorbent article 110. Specifically, in the example of Fig. 4, in the coating regions 440 of the adhesive 61, the adhesive 61 is applied in the longitudinal direction of the absorbent article with a width dimension of 15 mm and at an interval of 35 mm to be in two strips each having a blind shape. In the example of Fig. 4, preferably, a longitudinal dimension of the coating regions 440 of the adhesive 61 is longer than at least a length in a longitudinal direction of the endothermic material 51.

Moreover, in the absorbent article 110 according to the present embodiment, the coating regions 440 of the adhesive 61 are regions not facing the arrangement region 225 of the endothermic material 51 in a thickness direction of the absorbent article 110. In other words, the whole surfaces of the coating regions 440 of the adhesive 61 are the regions not facing the arrangement region 225 of the endothermic material 51.

According to the absorbent article 110 of the second embodiment, the whole surfaces of the coating regions 440 of the adhesive 61 are the regions not facing the arrangement region 225. Accordingly, there is almost no influence on the adhesive strength of the adhesive 61 in the coating regions 440 even when the temperature of the body fluid is reduced by the endothermic material 51.

Thus, according to the absorbent article 110 of the present embodiment, deterioration of the adhesive strength of the adhesives 61 can be prevented, and thus the absorbent article 110 on the underwear can less likely to be displaced or twisting.

In this way, the absorbent article 110 of the second embodiment of the present invention can suppress the stuffiness that the wearer feels when the body fluid such as menstrual blood is excreted, and can be less likely to be displaced or twist on underwear due to deterioration of the adhesive strength of the adhesive 61.

### (Absorbent Article According to Third Embodiment of the Present Invention)

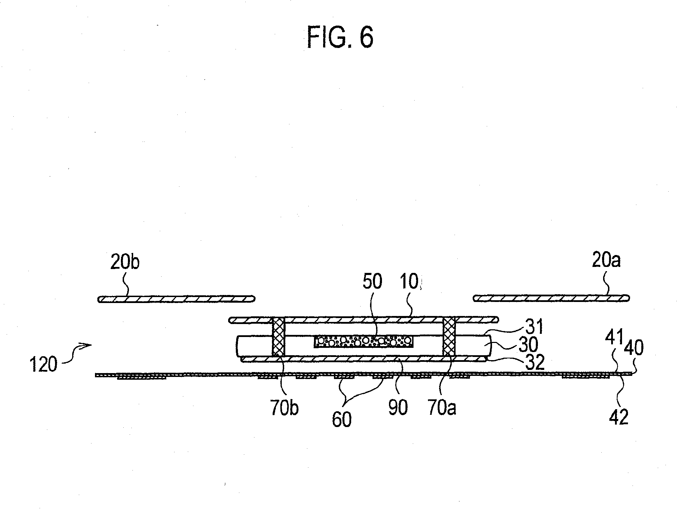

With reference to Fig. 6, a description will be given of an absorbent article according to a third embodiment of the present invention focusing on differences from the absorbent article 100 according to the first embodiment of the present invention. Fig. 6 is a sectional view of the absorbent article 120 according to the third embodiment of the present invention.

As shown in Fig. 6, in the absorbent article 120 according to the present embodiment, a hydrophobic sheet 90 is disposed between the back sheet 40 and the absorber 30. The absorbent article 120 according to the present embodiment has the same configuration as that of the absorbent article 100 according to the first embodiment except for the provision of the hydrophobic sheet 90.

Specifically, as shown in Fig. 6, in the absorbent article 120 according to the present embodiment, the hydrophobic sheet 90 is disposed on the surface 32 of the absorber 30 on the back sheet 40 side.

Moreover, preferably, an area in a planar direction of the hydrophobic sheet 90 is larger than at least an area of the arrangement region 225 of the endothermic material 50. The area in the planar direction of the hydrophobic sheet 90 may be approximately equal to the area of the absorber 30.

Any material can be used without particular limitation for the hydrophobic sheet 90, as long as the material can suppress reduction in temperature of the adhesive 60 disposed in the back sheet 40 due to the body fluid having its temperature reduced by the endothermic material 50. Examples of the material of the hydrophobic sheet 90 include liquid impermeable films and hydrophobic nonwoven fabric sheets.

When a film is used as the hydrophobic sheet 90, the body fluid having its temperature reduced by the endothermic material 50 does not come into contact with the back sheet 40. Accordingly, the temperature of the back sheet 40 and that of the adhesive 60 are less likely to reduce.

The number of the films used as the hydrophobic sheet 90 may be 1, or may be several by layering the films. When several films are layered, in the absorbent article 120, a space is provided between the surface 32 of the absorber 30 on the back sheet 40 side and the surface 41 of the back sheet 40 on the absorber 30 side. Thereby, thermal conductivity reduces and thus the temperature of the back sheet 40 and that of the adhesive 60 are less likely to reduce. However, from a viewpoint of reducing cost, it is preferable that a film be three-dimensionally formed to thereby provide a space between the surface 32 and the surface 41. In this case, projections and depressions may be formed on the film by projections and depressions embossing, etc., or projections, depressions and openings may be formed on a film by perforation. The materials and forms of the back sheet 40 shown in the first embodiment may be used as the material of the hydrophobic sheet 90.

When the material of the hydrophobic sheet 90 is a hydrophobic nonwoven fabric sheet, a space (space between fibers) is provided between the surface 32 and the surface 41. Thereby, thermal conductivity reduces so that the temperature of the back sheet 40 and that of the adhesive 60 are less likely to reduce. Further, it is preferable to use a bulky hydrophobic nonwoven fabric sheet which provides a larger space. Methods for forming this space include the three-dimensionally forming method mentioned above.

Moreover, it is preferable to use as the material of the hydrophobic sheet 90 a nonwoven fabric sheet rather than a film since the nonwoven fabric sheet does not excessively increase rigidity of the absorbent article 120. Specifically, materials not subjected to hydrophilic treatment or materials subjected to water-repellent treatment may be selected from the materials and forms of the top sheet 10 shown in the first embodiment.

In the absorbent article 120 according to the third embodiment of the present invention, the hydrophobic sheet 90 is disposed between the back sheet 40 and the absorber 30.

Accordingly, in the absorbent article 120 according to the present embodiment, the hydrophobic sheet 90 blocks the body fluid having its temperature reduced by the endothermic material 50. Therefore, it is possible to suppress reduction in temperature of the back sheet 40 and that of the adhesive 60 by the body fluid.

Thus, according to the absorbent article 120 of the third embodiment of the present invention, deterioration of the adhesive strength of the adhesive 60 can be suppressed. Accordingly, the absorbent article 120 is less likely to be displaced or twist on the underwear.

### (Other Embodiments)

While the sanitary napkin has been described as an example of the absorbent article in the above-mentioned embodiments, the present invention will not be limited to the sanitary napkin, and can also be applied to feminine absorbent articles such as panty liners. Further, the absorbent article can also be applied to incontinence pads and diapers.

As mentioned above, while the present invention has been described in detail using the above-mentioned embodiments, it should be obvious for those skilled in the art that the present invention should not be limited to the embodiments described herein. The present invention can be implemented as modifications and modified aspects, without departing from the spirit and scope of the present invention defined by the description of the scope of claims. Accordingly, the description herein is aimed at describing an example, and it does not have any restrictive sense to the present invention. Further, embodiments and modifications of the present invention can be combined.

Whole contents of Japan Patent Application No. 2008-140054 (filed on May 28, 2008) are incorporated herein by reference.

### Industrial Applicability

As described above, according to the present invention, it is applicable for an absorbent article and a sanitary napkin capable of suppressing stuffiness that the wearer feels when a body fluid such as menstrual blood or urine is excreted, the absorbent article being also less likely to be displaced or twist on underwear while being worn.

## Claims

1. An absorbent article comprising:
a liquid permeable top sheet;
a liquid impermeable back sheet; and
an absorber interposed between the top sheet and the back sheet, wherein
a coating region of an adhesive is formed in a planar direction on a surface of the back sheet facing clothing,
an endothermic material is disposed inside the absorber, and
a region not facing an arrangement region of the endothermic material in a thickness direction of the absorbent article, is formed at least in a part of the coating region of the adhesive.

2. The absorbent article according to claim 1, wherein the endothermic material is disposed inside the absorber on a top sheet side.

3. The absorbent article according to claim 1, wherein a hydrophobic sheet is disposed between the back sheet and the absorber.

4. A sanitary napkin comprising:
a liquid permeable top sheet;
a liquid impermeable back sheet; and
an absorber interposed between the top sheet and the back sheet, wherein
a coating region of an adhesive is formed in a planar direction on a surface of the back sheet facing clothing,
an endothermic material is disposed inside the absorber, and
a region not facing an arrangement region of the endothermic material in a thickness direction of the absorbent article, is formed at least in a part of the coating region of the adhesive.

5. The sanitary napkin according to claim 4, further comprising a wing extending in a width direction, wherein
in the sanitary napkin, a front region, a central region, and a rear region are provided continuously in a longitudinal direction of the absorber,
the endothermic material is disposed inside the absorber in an arrangement region including at least a central region of the absorbent article, and
a regionin a longitudinal direction of the central region, is a region in a longitudinal direction of the wing.

6. The sanitary napkin according to claim 4, wherein
in the sanitary napkin, an embossed groove is formed in both side portions in a width direction of the absorber,
the embossed groove is formed along a longitudinal direction of the sanitary napkin, and
the endothermic material is interposed between the embossed grooves.
